# EUROPÄISCHE PATENTSCHRIFT

(11) **EP 1 840 223 B1**
(45) Veröffentlichungstag und Bekanntmachung des Hinweises auf die Patenterteilung: **18.11.2009**
(21) Anmeldenummer: 06006213.0
(22) Anmeldetag: 25.03.2006
(51) Int. Cl.: C12Q 1/68

(54) **Verfahren zur mikroskopischen Ortsbestimmung eines ausgewählten, intrazellulären DNA-Abschnitts bekannter Nukleotidsequenz**
Method for microscopically localizing a selected intracellular stretch of known DNA
Méthode pour localiser une portion intracellulaire choisie d'un ADN connu à l'aide d'un microscope

(43) Veröffentlichungstag der Anmeldung: 03.10.2007
(73) Patentinhaber: Ruprecht-Karls-Universität Heidelberg, 69117 Heidelberg (DE)
(72) Erfinder: Hausmann, Michael, Prof. Dr., 76071 Ludwigshafen (DE); Cremer, Christoph, Prof. Dr. Dr., 69126 Heidelberg (DE)
(74) Vertreter: Rudolph, Ulrike

(56) Entgegenhaltungen:
- WO-A-95/03428
- WO-A-98/37231
- WO-A1-O3/091455
- SCHWARZ-FINSTERLE ET AL: "COMBO-FISH for focussed fluorescence labelling of gene domains: 3D-analysis of the genome architecture of abl and bcr in human blood cells" CELL BIOLOGY INTERNATIONAL, ACADEMIC PRESS, GB, Bd. 29, Nr. 12, Dezember 2005 (2005-12), Seiten 1038-1046, XP005197826 ISSN: 1065-6995
- HAUSMANN MICHAEL ET AL: "COMBO-FISH: Specific labeling of nondenatured chromatin targets by computer-selected DNA oligonucleotide probe combinations." BIOTECHNIQUES, Bd. 35, Nr. 3, September 2003 (2003-09), Seiten 564-577, XP008064869 ISSN: 0736-6205
- WINKLER R ET AL: "Labelling quality and chromosome morphology after low temperature FISH analysed by scanning far-field and near-field optical microscopy." JOURNAL OF MICROSCOPY. JAN 2003, Bd. 209, Nr. Pt 1, Januar 2003 (2003-01), Seiten 23-33, XP002383502 ISSN: 0022-2720
- ANONYMOUS: "15th Annual Meeting of the German-Society-for-Cytometry, Leipzig, GERMANY, October 19 -22, 2005" CELL PROLIFERATION, Bd. 38, Nr. 4, August 2005 (2005-08), Seiten 175-214, XP002383503 & 15TH ANNUAL MEETING OF THE GERMAN-SOCIETY-FOR-CYTOMETRY; LEIPZIG, GERMANY; OCTOBER 19 -22, 2005 ISSN: 0960-7722
- HILDENBRAND GEORG ET AL: "Nano-sizing of specific gene domains in intact human cell nuclei by spatially modulated illumination light microscopy" BIOPHYSICAL JOURNAL, Bd. 88, Nr. 6, Juni 2005 (2005-06), Seiten 4312-4318, XP008064833 ISSN: 0006-3495
- TUMBAR T ET AL: "Interphase movements of a DNA chromosome region modulated by VP16 transcriptional activator." NATURE CELL BIOLOGY. FEB 2001, Bd. 3, Nr. 2, Februar 2001 (2001-02), Seiten 134-139, XP002383504 ISSN: 1465-7392

### SEQUENCE LISTING

<110> Ruprecht-Karls-Universität Heidelberg
   Hausmann, Michael
   Cremer, Christoph
<120> verfahren zur mikroskopischen Ortsbestimmung eines ausgewählten intrazellulären, DNA-Abschnitts bekannter Nukleotidsequenz
<130> cre 5
<160> 69
<170> PatentIn version 3.3
<210> 1
   <211> 21
   <212> DNA
   <213> human
<220>
   <221> gene
   <222> (1)..(21)
<400> 1
   aacctttctt ttctcttcca a 21
<210> 2
   <211> 20
   <212> DNA
   <213> Human
<220>
   <221> gene
   <222> (1)..(20)
<400> 2
   ccaaaagaag aaagaaggtc 20
<210> 3
   <211> 19
   <212> DNA
   <213> Human
<220>
   <221> gene
   <222> (1)..(19)
<400> 3
   ttaaagagag gagaaggtg 19
<210> 4
   <211> 25
   <212> DNA
   <213> Human
<220>
   <221> gene
   <222> (1)..(25)
<400> 4
   gtgagagaga aagagaaaga gagtg 25
<210> 5
   <211> 21
   <212> DNA
   <213> Human
<220>
   <221> Gene
   <222> (1)..(21)
<400> 5
   tattctttcc ttttctttta c 21
<210> 6
   <211> 19
   <212> DNA
   <213> Human
<220>
   <221> Gene
   <222> (1)..(19)
<400> 6
   ttgagaggga aagggaata 19
<210> 7
   <211> 21
   <212> DNA
   <213> Human
<220>
   <221> Gene
   <222> (1)..(21)
<400> 7
   atgggagagg aggagaagat g 21
<210> 8
   <211> 19
   <212> DNA
   <213> Human
<220>
   <221> Gene
   <222> (1)..(19)
<400> 8
   agttcctttt cctttccat 19
<210> 9
   <211> 21
   <212> DNA
   <213> Human
<220>
   <221> Gene
   <222> (1)..(21)
<400> 9
   gatccttcct ttctcccctg t 21
<210> 10
   <211> 35
   <212> DNA
   <213> Human
<220>
   <221> Gene
   <222> (1)..(35)
<400> 10
   ctgaaggaag gaaggaggga ggaaggaagg aagca 35
<210> 11
   <211> 22
   <212> DNA
   <213> Human
<220>
   <221> Gene
   <222> (1)..(22)
<400> 11
   gtagagggaa gggagaaagg tg 22
<210> 12
   <211> 19
   <212> DNA
   <213> Human
<220>
   <221> Gene
   <222> (1) .. (19)
<400> 12
   tcaaaagaag gagaagatg 19
<210> 13
   <211> 21
   <212> DNA
   <213> Human
<220>
   <221> Gene
   <222> (1)..(21)
<400> 13
   tgtcttcctt tcttcctcca t 21
<210> 14
   <211> 23
   <212> DNA
   <213> Human
<220>
   <221> Gene
   <222> (1)..(23)
<400> 14
   tgtctctctc tctcctcccc ccc 23
<210> 15
   <211> 30
   <212> DNA
   <213> Human
<220>
   <221> Gene
   <222> (1)..(30)
<400> 15
   cacccctctc tctccttctc tcttttctgt 30
<210> 16
   <211> 19
   <212> DNA
   <213> Human
<220>
   <221> Gene
   <222> (1)..(19)
<400> 16
   tgctcccctc cctcctcac 19
<210> 17
   <211> 20
   <212> DNA
   <213> Human
<220>
   <221> Gene
   <222> (1)..(20)
<400> 17
   cctcctcttc tctccctccc 20
<210> 18
   <211> 16
   <212> DNA
   <213> Human
<220>
   <221> Gene
   <222> (1)..(16)
<400> 18
   tcttcctttt ctctct 16
<210> 19
   <211> 15
   <212> DNA
   <213> Human
<220>
   <221> Gene
   <222> (1) .. (15)
<400> 19
   ctttccctcc ctctt 15
<210> 20
   <211> 15
   <212> DNA
   <213> Human
<220>
   <221> Gene
   <222> (1)..(15)
<400> 20
   ccttcttctt tcccc 15
<210> 21
   <211> 16
   <212> DNA
   <213> Human
<220>
   <221> Gene
   <222> (1)..(16)
<400> 21
   cctcctcttt tctcct 16
<210> 22
   <211> 20
   <212> DNA
   <213> Human
<220>
   <221> Gene
   <222> (1)..(20)
<400> 22
   ttccttcctt ccctctttcc 20
<210> 23
   <211> 24
   <212> DNA
   <213> Human
<220>
   <221> Gene
   <222> (1)..(24)
<400> 23
   ctcttttctt ttcttttctt tctt 24
<210> 24
   <211> 16
   <212> DNA
   <213> Human
<220> Gene
   <221> Gene
   <222> (1)..(16)
<400> 24
   ctccttcctt tctctc 16
<210> 25
   <211> 18
   <212> DNA
   <213> Human
<220>
   <221> Gene
   <222> (1)..(18)
<400> 25
   ttctctttct tctcttct 18
<210> 26
   <211> 15
   <212> DNA
   <213> Human
<220>
   <221> Gene
   <222> (1) .. (15)
<400> 26
   tttctctttc ctcct 15
<210> 27
   <211> 17
   <212> DNA
   <213> Human
<220>
   <221> Gene
   <222> (1) .. (17)
<400> 27
   tctttccctt tccctcc 17
<210> 28
   <211> 17
   <212> DNA
   <213> Human
<220>
   <221> Gene
   <222> (1)..(17)
<400> 28
   cctcccttcc cccttcc 17
<210> 29
   <211> 17
   <212> DNA
   <213> Human
<220>
   <221> Gene
   <222> (1)..(17)
<400> 29
   ctccttttcc cttccct 17
<210> 30
   <211> 19
   <212> DNA
   <213> Human
<220>
   <221> Gene
   <222> (1)..(19)
<400> 30
   ccctttccct ttctctccc 19
<210> 31
   <211> 18
   <212> DNA
   <213> Human
<220>
   <221> Gene
   <222> (1) .. (18)
<400> 31
   cccccttcct cccctttc 18
<210> 32
   <211> 17
   <212> DNA
   <213> Human
<220>
   <221> Gene
   <222> (1)..(17)
<400> 32
   tctctctccc tcccctc 17
<210> 33
   <211> 22
   <212> DNA
   <213> Human
<220>
   <221> Gene
   <222> (1)..(22)
<400> 33
   tcttctctcc tcttctctcc tc 22
<210> 34
   <211> 18
   <212> DNA
   <213> Human
<220>
   <221> Gene
   <222> (1)..(18)
<400> 34 18
   cttcctccct tctcctcc 18
<210> 35
   <211> 18
   <212> DNA
   <213> Human
<220>
   <221> Gene
   <222> (1) .. (18)
<400> 35 18
   ccctcccccc tccctccc 18
<210> 36
   <211> 16
   <212> DNA
   <213> Human
<220>
   <221> Gene
   <222> (1)..(16)
<400> 36
   ccccttcctc ctccct 16
<210> 37
   <211> 16
   <212> DNA
   <213> Human
<220>
   <221> Gene
   <222> (1)..(16)
<400> 37 16
   cctccttctc ccctct 16
<210> 38
   <211> 21
   <212> DNA
   <213> Human
<220>
   <221> Gene
   <222> (1)..(21)
<400> 38 21
   cctctctccc tttttcttct t 21
<210> 39
   <211> 17
   <212> DNA
   <213> Human
<220>
   <221> Gene
   <222> (1)..(17)
<400> 39
   agaagggaag ggaggga 17
<210> 40
   <211> 18
   <212> DNA
   <213> Human
<220>
   <221> Gene
   <222> (1)..(18)
<400> 40
   agaggggaag ggaggagg 18
<210> 41
   <211> 16
   <212> DNA
   <213> Human
<220>
   <221> Gene
   <222> (1)..(16)
<400> 41
   ctctctttct ctcccc 16
<210> 42
   <211> 18
   <212> DNA
   <213> Human
<220>
   <221> Gene
   <222> (1)..(18)
<400> 42
   cctctctcct ctccttcc 18
<210> 43
   <211> 19
   <212> DNA
   <213> Human
<220>
   <221> Gene
   <222> (1)..(19)
<400> 43
   aggagggagg aagagaggg 19
<210> 44
   <211> 17
   <212> DNA
   <213> Human
<220>
   <221> Gene
   <222> (1)..(17)
<400> 44
   ccccctctcc ttctcct 17
<210> 45
   <211> 22
   <212> DNA
   <213> Human
<220>
   <221> Gene
   <222> (1)..(22)
<400> 45
   ggggaggaga gaaaagaagg ag 22
<210> 46
   <211> 17
   <212> DNA
   <213> Human
<220>
   <221> Gene
   <222> (1)..(17)
<400> 46 17
   cctccctttc ctctccc 17
<210> 47
   <211> 22
   <212> DNA
   <213> Human
<220>
   <221> Gene
   <222> (1)..(22)
<400> 47
   aggagaggag agagagggaa ga 22
<210> 48
   <211> 22
   <212> DNA
   <213> Human
<220>
   <221> Gene
   <222> (1) .. (22)
<400> 48
   ccttccttcc cccctctccc ct 22
<210> 49
   <211> 21
   <212> DNA
   <213> Human
<220>
   <221> Gene
   <222> (1)..(21)
<400> 49
   gggggagggg aagaaggagg g 21
<210> 50
   <211> 16
   <212> DNA
   <213> Human
<220>
   <221> Gene
   <222> (1) .. (16)
<400> 50
   ttcctttctc ctcctc 16
<210> 51
   <211> 17
   <212> DNA
   <213> Human
<220>
   <221> Gene
   <222> (1)..(17)
<400> 51
   ctcccttttc tcttctt 17
<210> 52
   <211> 19
   <212> DNA
   <213> Human
<220>
   <221> Gene
   <222> (1)..(19)
<400> 52
   ctttctcccc ttctccctc 19
<210> 53
   <211> 16
   <212> DNA
   <213> Human
<220>
   <221> Gene
   <222> (1)..(16)
<400> 53
   aaggagaaaa ggagga 16
<210> 54
   <211> 16
   <212> DNA
   <213> Human
<220>
   <221> Gene
   <222> (1)..(16)
<400> 54
   gaggggagaa gggagg 16
<210> 55
   <211> 15
   <212> DNA
   <213> Human
<220>
   <221> Gene
   <222> (1)..(15)
<400> 55
   cctcctctct ctccc 15
<210> 56
   <211> 19
   <212> DNA
   <213> Human
<220>
   <221> Gene
   <222> (1)..(19)
<400> 56
   gggaaggaga agaggaagg 19
<210> 57
   <211> 17
   <212> DNA
   <213> Human
<220>
   <221> Gene
   <222> (1)..(17)
<400> 57
   cccctcctcc ttcctct 17
<210> 58
   <211> 15
   <212> DNA
   <213> Human
<220>
   <221> Gene
   <222> (1)..(15)
<400> 58
   agaggaagag aagaa 15
<210> 59
   <211> 22
   <212> DNA
   <213> Human
<220>
   <221> Gene
   <222> (1)..(22)
<400> 59
   ccttctttcc tctctccttc cc 22
<210> 60
   <211> 16
   <212> DNA
   <213> Human
<220>
   <221> Gene
   <222> (1)..(16)
<400> 60
   ccctttctcc tccccc 16
<210> 61
   <211> 28
   <212> DNA
   <213> Human
<220>
   <221> Gene
   <222> (1)..(28)
<400> 61
   tctcttttcc tttctcttcc ccctcctc 28
<210> 62
   <211> 15
   <212> DNA
   <213> Human
<220>
   <221> Gene
   <222> (1)..(15)
<400> 62
   aaaggaagag aagaa 15
<210> 63
   <211> 16
   <212> DNA
   <213> Human
<220>
   <221> Gene
   <222> (1)..(16)
<400> 63
   cccctttccc ttccct 16
<210> 64
   <211> 19
   <212> DNA
   <213> Human
<220>
   <221> Gene
   <222> (1)..(19)
<400> 64
   ccccccttcc tctcctctt 19
<210> 65
   <211> 25
   <212> DNA
   <213> Human
<220>
   <221> Gene
   <222> (1)..(25)
<400> 65
   gggggggagg gaagagagaa agaga 25
<210> 66
   <211> 16
   <212> DNA
   <213> Human
<220>
   <221> Gene
   <222> (1)..(16)
<400> 66
   gaagagaggg agaaag 16
<210> 67
   <211> 17
   <212> DNA
   <213> Human
<220>
   <221> Gene
   <222> (1)..(17)
<400> 67
   gagaaggaag gagagag 17
<210> 68
   <211> 16
   <212> DNA
   <213> Human
<220>
   <221> Gene
   <222> (1)..(16)
<400> 68
   cctctttcct cctctc 16
<210> 69
   <211> 17
   <212> DNA
   <213> Human
<220>
   <221> Gene
   <222> (1)..(17)
<400> 69
   tccttccctc cccctct 17

## Beschreibung

Die Erfindung betrifft ein Fluoreszenz-in-situ-Hybridisierungs (FISH)-Verfahren zur mikroskopischen Analyse der Lokalisation eines ausgewählten intrazellulären, nativen Genomabschnitts mit bekannter Nukleotidsequenz - im folgenden "Target-DNA" - , insbesondere eines Gens innerhalb eines (bzw. auf einem) Chromosom(s).

Im Stand der Technik ist die spezifische Markierung von DNA-Regionen ("Target-DNAs") in Zellkernen und auf Chromosomen mit Hilfe von spezifischen DNA-, RNA- oder PNA-Sonden bekannt. Bei den Sonden handelt es sich um Einzelstrangmolekülketten, die durch molekularbiologische Vervielfältigungsverfahren wie z.B. Klonierung oder Polymerasekettenreaktion hergestellt werden, und die entsprechende komplementäre Zielsequenzen in einem Genom besitzen. Üblicherweise werden solche Sonden aus BAC-, Cosmid- oder YAC-Klonen hergestellt, die geeignet kombiniert werden, oder die durch molekularbiologische Verfahren an die Zielsequenz angepasst werden. Diese BAC-, Cosmid- oder YAC-Sonden legen die Größe und den Ort der Markierung fest. Die Länge dieser Sonden bzw. der komplementären Sequenzen stimmt jedoch oft nicht exakt mit der gewünschten zu markierenden DNA-Region überein, sondern überschreitet, insbesondere bei kleinen Genen, häufig um ein Mehrfaches diese Target-DNA. Ein Beispiel hierfür stellt das für die Tumordiagnostik und -therapie relevante Her2/neu Gen auf Chromosom 17 dar. Dieses Gen hat eine Länge von ca. 44 kb. Alle für die Markierung dieses Gens allgemein zur Verfügung stehenden BAC-Klone haben eine Länge in der Größenordnung von ca. 100 kb. Bei der Markierung von Gen Her2/neu durch Fluoreszenz-in-situ-Hybridisierung kommt es deshalb häufig zu einer zusätzlichen Markierung benachbarter Gene, wie z.B. des Gens Grb7. Damit ist eine differenzierte mikroskopische Einzelanalyse der Veränderungen des Einzelgens Her2/neu ohne komplexe molekularbiologische Modifikationen der Sonden nicht möglicht.
Ein ähnliches Problem besteht bei der experimentellen Bestimmung der Lage von Bruchpunkten in Chromosomen, die in Translokationen involviert sind. Solche Bruchpunkte können durch sich überlappende BAC-Klone zwar bis auf eine Region von wenigen 10 kb eingegrenzt werden. Eine noch genauere Ortsbestimmung bzw. Lokalisation erfordert dann aber Sequenzierverfahren oder PCR-Verfahren, die experimentell keine Aussage mehr zur Einzelzelle erlauben und zudem relativ arbeitsaufwändig sind. Hier ist es wünschenswert, diesen Arbeitsaufwand zu reduzieren, indem die Markierungsregionen kleiner und genauer definierbar werden.
Fluoreszenzmarkierungsverfahren von Genomregionen in Zellkernen und auf Chromosomen mit spezifischen DNA-, RNA-, PNA-Sonden werden in der Literatur üblicherweise als Fluoreszenz-in-situ-Hybridisierung (FISH) bezeichnet. Zur Markierung werden in diese Sonden Reportermoleküle eingebunden, die beispielsweise eine hohe Affinität zu entsprechenden Fluorochromkomplexen besitzen. Es können aber auch bestimmte Fluorochromkomplexe direkt in die Sondenmoleküle eingebaut werden, ggf. über einen Linker geeigneter Länge. Das zur Verfügung stehende Farbspektrum der Fluorochrome reicht über das sichtbare Spektrum bis ins Infrarote. Neben dem Emissions-Spektrum kann auch die Lebensdauer der Fluoreszenzemission als Parameter genutzt werden. Die Eigenschaften Absorptionsspektrum, Emissionsspektrum und Fluoreszenzlebensdauer werden als spektrale Signatur bezeichnet. Neben den fluoreszenten Markermolekülen sind weitere Signaturen bekannt, die eine spezifische Erkennung der markierten Genomregion ermöglichen, z.B. solche, die in der atomaren Kraftmikroskopie(Gold-, Silbermoleküle) oder in der Magnetresonanztomographie (paramagnetische Nanopartikel) eingesetzt werden.
Die Detektion der markierten Genomzielregionen erfolgt üblicherweise mittels FernfeldMikroskopie (z.B. Epifluoreszenzmikroskopie, konfokale Laser-ScanningMikroskopie, Wellenfeld-/SMI-Mikroskopie, Fluoreszenzkorrelationsmikroskopie, mit oder ohne axialtomographische Ergänzungstechniken, 4Pi-Mikroskopie etc.), mittels Nahfeldmikroskopie (AFM, SNOM etc.), mittels Magnetresonanzverfahren oder mittels anderer Verfahren zur ortsgenauen Bestimmung der Marker.
Die meisten bei FISH bisher eingesetzten Hybridisierungsprotokolle gehen davon aus, dass als Voraussetzung für diesen Prozess nicht nur die Sonde, sondern auch die Target-DNA, die nativ häufig in Doppelstrangform vorliegt, vollständig in eine einzelsträngige Form überführt werden muss (Denaturierung). Für die Denaturierung werden entweder chemische Behandlungsmethoden (z.B. unter Verwendung einer entsprechend hohen Konzentration des Lösungsmittels Formamid oder anderer chaotroper Substanzen), enzymatische und/oder thermische Behandlungsmethoden (z.B. Aufheizen auf Temperaturen über 70°C) vorgeschrieben bzw. vorgeschlagen. Die anschließend notwendigerweise herbeigeführte Renaturierung führt dann zur Bildung hybrider Doppelstränge aus Sonde und der Target-DNA (Standard-FISH).
Es ist ferner bekannt, dass einzelsträngige Sonden, in denen das Zucker-Phosphat-Rückgrat durch eine Polyamidkette ersetzt wurde (sogenannte PNA-Sonden), sich in einer sequenzspezifischen Weise mit einzel- oder doppelsträngiger DNA zu einer neuen zwei- oder dreisträngigen Formation verbinden können (PNA-DNA). Diese Erkenntnis wurde zur Visualisierung bestimmter repetitiver Sequenzen durch FISH experimentell realisiert (PNA-FISH). Aufgrund der durch den Einsatz von PNA bewirkten Eliminierung der elekrostatischen Abstoßungskräfte zwischen Target-DNA und Sonden-PNA ist dieses Verfahren mit weitgehend beliebigen Basensequenzen und sogar in Gegenwart hoher Konzentrationen an Formamid durchführbar; es hat jedoch den Nachteil eines erheblichen Aufwandes bei der Synthese der PNA-Sonden.
Aus der WO 03091455 (Genomics, Inc ) ist eine sogenannte "zweiarmige Nukleinsäuresonde" (Two-Arm Nucleic Acid Probe) bekannt, die aus einem Polynukleotid besteht, das an eine Target-Nukleinsäure binden kann, wobei das eine Ende dieses Polynukleotis mit der Target-Nukleinsäure über Hoogsteen-Bindungen einen Doppelstrang bildet, während das andere Ende dieses Polynukleotids mit derselben Target-Nukleinsäure- jedoch an einer anderen Stelle - über Watson-Crick-Bindungen eine Tripplehelix bildet, und wobei die Nukleotidsequenz zwischen diesen beiden Enden des Polynukleotids eine übliche Linkersequenz ist. Die Spezifität der Sonde wird durch die Kombination von Hoogsteen- und Watson-Crick-Bindung gewährleistet und für den Bindungsvorgang wird die Target DNA üblicherweise denaturiert.

Hinsichtlich der Frage nach dem Hybridisierungsmechanismus ging die Fachwelt bislang davon aus, dass die in jedem Genom von Natur aus vorhandenen einzelsträngigen DNA-Abschnitte für eine in-situ-Hybridisierung zahlenmäßig nicht ausreichend sind. Andererseits ist in der Publikation von Winkler et al. (Journal of Microscopy, 2003) beschrieben, dass bestimmte DNA-Sonden auch dann an chromosomale Zielsequenzen spezifisch hybridisieren, wenn keine Denaturierung des Targets vorgenommen wurde. Diese Ergebnisse werden durch Dobrucki J. und Darzynkiewicz Z. (2001) Micron, Bd. 32, S.645-652, unterstützt.
Ferner ist in der DE 198 06 962 ein in-situ-Hybridisierungsverfahren beschrieben, das auf Denaturierung verzichtet und einzelsträngige Homopurin- oder Homopyrimidinsequenzen als Sonden an die doppelsträngige Target-DNA via Hoogsteen Bindungen hybridisiert. Die einzelsträngigen Homopurin- oder Homopyrimidinsonden binden mit den Basen der doppelsträngigen Duplex-Nukleinsäuresequenzen und formen dabei eine dreisträngige Triple-Helix. Damit sind die Voraussetzungen für FISH an lebenden oder vitalen Zellen geschaffen, denn diese Sonden können durch Mikroinjektionsverfahren in solche Zellen eingebracht werden und lagern sich dann spezifisch an die komplementären Abschnitte der Duplex-DNA an, ohne dass eine weitere Behandlung der Zellen erfolgen muß. Alternativ sind bisher *in-vivo*-Markierungsverfahren bekannt für: (i) Nukleoli mit Hilfe einer Fluoreszenz in situ Hybridisierung von RNA-Proben (RNA-FISH); (ii) Zentromerregionen mit einem zentromerspezifischen Protein, das mit Hilfe eines gekoppelten "Green Fluorescent Protein" (GFP) visualisiert wurde; (iii) gewisse "Homogeneously Staining Regions" (HSR) mit einem Lac Operon-spezifischen GFP-gekoppelten Protein; und (iv) ganze Chromosomenterritorien unter Ausnutzung von Replikationsmechanismen.
Auch aus der US 5,176,996 ist bekannt, dass definierte einzelsträngige Sonden-Oligonukleotide synthetisiert werden können, die spezifisch an ausgewählte Doppelstrang-DNA-Sequenzen binden, wobei ein Tripelstrang entsteht, so dass Genfunktionen wie die Proteinsynthese blockiert werden. Die beschriebenen Sonden-Oligonukleotide weisen üblicherweise eine Länge von mehr als 20 Nukleotiden auf und tragen keine Markermoleküle.
Aus der Druckschrift Hausmann, M. et al. 2003 (BIOTECHNIOUES, Bd.35. Nr. 3, S. 564-577) , ist ein Fluoreszenz-in-situ-Hybridisierungsverfahrens Verfahren bekannt, das zur mikroskopischen Analyse der Lokalisation eines ausgewählten, intrazellulären und nativen Genomabschnitts mit bekannter Nukleotidsequenz unter Ausbildung einer Dreifach-Helixstruktur in nicht-denaturierten, potentiell lebendigen Zellen vorgesehen ist. Die dabei verwendeten Sonden bestehen aus Homopurin- und/oder Homopyrimidin-Sequenzen, welche unter Ausbildung von Hoogsteen Basenpaarungen miteinander hybridisieren.

Keines der bekannten FISH-Verfahren oder alternativen *in*-*vivo*-Markierungsverfahren erlaubt es jedoch, ganz beliebige und gegebenenfalls auch sehr kleine Genomregionen nukleotidgenau und praktisch (nahezu) ohne Veränderungen der nativen funktionellen Organisation spezifisch in vitalen Zellen in situ zu markieren.
Sowohl in der Grundlagenforschung als auch insbesondere in der medizinischen Diagnostik besteht jedoch Bedarf an einem Verfahren, mit dem nukleotidgenau selbst kleine Genomregionen dargestellt werden können und im Idealfall zudem Mutationen, Amplifikationen oder Deletionen derselben. Dabei soll das Genommaterial möglichst ohne oder mit nur geringer Vorbehandlung markierbar und analysierbar sein, um Schäden der nativen Strukturen zu vermeiden. Dies bedeutet, daß das Genommaterial und damit folglich auch die betreffende(n) Zelle(n) in vivo oder in einem dem in-vivo-Zustand nahe kommenden (vitalen) Zustand erhalten sein bzw. bleiben müssen. Alle bisher bekannten in-situ-Hybridisierungsverfahren, bei denen von einem vital konservierten Target-Genom gesprochen werden kann, erlauben jedoch keine FISH beliebiger spezifischer, kleiner Genomabschnitte, wie z.B. einzelner Gene oder tumorrelevanter Genomloci.

Der vorliegenden Erfindung liegt deshalb die Aufgabe zugrunde, ein **FISH-**Verfahren bereit zustellen, mit dem jede beliebige und unter Umständen auch noch sehr kleine und hinsichtlich ihrer Nukleotidsequenz bekannte Genomregionen nukleotidgenau und ohne oder allenfalls äußerst geringe Veränderungen der nativen strukturellen und funktionellen Organisation zu markieren, so daß mit Hilfe mikroskopischer Techniken eine Ortsbestimmung dieser Genomregion in bzw. auf dem Chromosom oder Genom möglich ist, und damit auch Folgemaßnahmen, die die Kenntnis der genauen Lokalisation eines Gens voraussetzen.

Eine Lösung dieser Aufgabe besteht in einem **FISH-**Verfahren der eingangs genannten Art, das durch die Art und Reihenfolge der folgenden Maßnahmen gekennzeichnet ist, nämlich dadurch, daß (1.) die Target-DNA anhand von (bekannten) Genomdatenbanken hinsichtlich (a) einer einzigen oder (b) mehrerer gleichartiger oder verschiedener Teilsequenzen analysiert wird, und solche Teilsequenzen ausgewählt werden, die alleine oder in Kombination ein einmaliges Muster innerhalb des Genoms darstellen, welches im Rest des Genoms außerhalb der Target-DNA nicht mehr innerhalb von beliebigen Genomabschnitten mit der Länge der Target-DNA vorkommen, das heißt in dieser speziellen Anordnung, in der sie innerhalb der Target-DNA vorliegt bzw. vorliegen, kommt bzw. kommen sie nicht auch im übrigen Genom vor, wobei jede Teilsequenz eine Länge von 8 bis 40 Nukleotiden aufweist, daß (2.) dass diese Teilsequenzen in entsprechende komplementäre Sondensequenzen umgeschrieben und als einzelsträngige Sondensequenzen bereitgestellt werden, die mit der/den Teilsequenz(en) der Target-DNA übereinstimmen oder komplementär dazu sind, und die über eine Watson-Crick-Bindung zur Hybridisierung an die - natürlicherweise wenigstens temporär (zeitweise, vorübergehend), z.B. während der Zellteilung - als zwei DNA-Einzelstränge der Target-DNA vorliegenden Teilsequenz(en) geeignet sind, daß (3.) diese Sondensequenzen mit gleichartigen und/ oder verschiedenartigen, mikroskopisch oder spektroskopisch oder durch Magnetresonanz detektierbaren Markermolekülen gekoppelt sind, wobei im Fall (1b) einer Kombination mehrerer Teilsequenzen bzw. Sondensequenzen und deren Kopplung mit verschiedenen Markermoleküle(n) jede Einheit aus Sondensequenz und Markermolekül(en) praktisch das gleiche Bindungsverhalten oder den gleichen Schmelzpunkt mit dem zu ihr komplementären Einzelstrang der Target-DNA aufweist wie jede der anderen Einheiten (das heißt mit anderen Worten: alle Einheiten aus Sondensequenz und Markermolekül(en) haben im wesentlichen das gleiche Bindungsverhalten, nämlich die gleiche Bindungsenergie und/oder Bindungskinetik, und/oder den gleichen Schmelzpunkt mit dem Einzelstrang des Target DNA-Abschnitts), daß (4.) diese Sondensequenz(en) mittels bekannter Verfahren unter Nichtanwendung eines Denaturierungsschrittes derart in die Zelle eingebracht und mit der Target-DNA zusammengebracht werden, daß sie mit den entsprechenden temporär (vorübergehend, zeitweise) als zwei DNA-Einzelstränge der Target-DNA vorliegenden Teilsequenz(en) der Target-DNA Ausbildung von Watson-Crick-Basenpaarung hybridisieren, daß (5.) die emittierten Markersignale detektiert werden, und daß (6.) anhand des Vorhandenseins und/oder der Intensität und/oder des gleichzeitigen Auftretens von verschiedenen Markersignalen der Ort der Target-DNA auf dem Genom, insbesondere dem Chromosom identifiziert wird.

Natürlicherweise temporär (vorübergehend, zeitweise) als zwei DNA-Einzelstränge liegt die (humane) DNA beispielsweise während der Zellteilung, insbesondere während der DNA-Verdopplung vor. Eine solche Situation der "natürlicherweise temporär vorliegenden Einzelstränge" kann der Fachmann bei Bedarf auch induzieren bzw. stimulieren, indem er beispielsweise die Zellteilung der betreffenden Zellen stimuliert bzw. induziert.

Der Begriff "Bindungsverhalten" steht hier und im folgenden für die Kombination "Bindungsenergie und Bindungskinetik und zahlenmäßiges Verhältnis von Purinen zu Pyrimidinen".

Der Vorteil dieses Verfahrens besteht darin, dass beliebige Genomregionen exakt fokussiert markiert werden und nicht, wie bei den bisher üblichen FISH-Verfahren, die Sonden durch die Größe der molekularbiologisch hergestellten BAC-, Cosmid- oder YAC-Klone und ihre Bindungsstellen bestimmt sind, so dass insbesondere Genomregionen von wenigen kb nur ungenau oder viel zu weit umfassend getroffen werden.

Da die Sonden je nach gewünschtem FISH Verfahren (Standard FISH mit Denaturierung der Ziel-DNA, FISH ohne Denaturierung, FISH gegen DNA-Einzelstränge oder Doppelstränge etc.) mit derselben Sequenz und zudem mit unterschiedlicher Orientierung synthetisiert werden können, kann dieses Verfahren hochgradig flexibel an das jeweilige Zielmaterial und dessen Qualität angepasst werden.

Das Verfahren wird vorzugsweise in vivo bzw. vital (d.h. dem in-vivo-Zustand nahe) durchgeführt, um ein Höchstmaß an Genauigkeit bei der Ortsbestimmung des darzustellenden Gens oder Gensegments zu gewährleisten.

Die Teilsequenz und damit auch die Sondensequenzen haben vorzugsweise eine Länge von 10 bis 40 Nukleotiden.

Als Markermoleküle werden insbesondere Fluoreszenzfarbstoffe, immunhistochemische Markermoleküle, fluoreszente oder luminiszente Nanopartikel, insbesondere Nanokristalle (Quantendots), und magnetische Nanopartikel vorgeschlagen, wobei auch Kombinationen verschiedener solcher Markermolekültypen zum Einsatz kommen können.

Eine Weiterbildung des Verfahrens, die insbesondere in der Tumordiagnostik vorteilhaft eingesetzt werden kann, ermöglicht den Nachweis von Punktmutationen und/oder Genom-Bruchpunkten und/oder äquivalenten Sequenzdefekten (Mikrodeletionen), und ist **dadurch gekennzeichnet, daß** in Schritt (1) des Basisverfahrens die Target-DNA hinsichtlich mehrerer (gleichartiger oder verschiedener) Teilsequenzen t₁-tₙ analysiert wird, daß in Schritt (3) die Sondensequenzen S₁-Sₙ mit verschiedenartigen, mikroskopisch oder spektroskopisch oder durch Magnetresonanz detektierbaren Markermolekülen gekoppelt sind, und daß in Schritt (6) die Abwesenheit der Markersignale einer oder mehrerer Sondensequenzen Sₓ bei gleichzeitiger Anwesenheit der Markersignale der Sondensequenzen Sₙ-Sₓ das Vorliegen einer Punktmutation oder eines Genombruchpunktes oder eines äquivalenten Sequenzdefektes anzeigt.

Insbesondere für dieses weiterentwickelte Verfahren können die Sondensequenzen so konstruiert werden, dass sie nicht mehr an die Target-DNA binden, sobald auch nur eine ihrer Basen nicht komplementär zur Zielsequenz ist. Damit ist es möglich, anhand des Fehlens des Signals einer bestimmten Sondensequenz in einem Sondengemisch eine Mutation zu entdecken. Auch Deletionen und insbesondere Mikrodeletionen können auf diese Weise aufgedeckt werden.

Bruchpunkte innerhalb der Target-DNA können mit diesem weiterentwickelten Verfahren ebenfalls erkannt und eingegrenzt werden. Sie werden dadurch angezeigt, dass die verschiedenen Sondensequenzen eines bereitgestellten Sondensets nicht alle an einem Genomort binden, sondern an zwei oder mehr voneinander beabstandeten Genomorten detektiert werden.

Die Sonden und/oder Sondensets werden mittels Standardverfahren der Biochemie, Molekularbiologie oder Zytogenetik in die Zielzellen eingebracht. Bevorzugt sind Diffusionsverfahren, Verfahren der Mikroinjektion, Verfahren der Elektroporation und Verfahren der Vesikel-aktivierten Membrantransvektion.

Zur Verwendung in dem FISH (fluorescence-in-situ-hybridization)-Verfahren wird ein Sondenset vorgeschlagen, das einen einzigen oder mehrere Sondentyp(en) Sₗ - Sₙ enthält, wobei jeder Sondentyp Sᵢ aus einem Oligonukleotid oder einem dazu äquivalenten Molekül (z.B. einem PNA-Oligomer) besteht, das eine Länge von 8 bis 40 Nukleotiden aufweist, und das komplementär zu oder identisch mit einer einsträngigen Teilsequenz **t**ᵢ eines intrazellulären, doppelsträngigen Genomabschnitts mit bekannter Nukleotidsequenz - im folgenden "Target-DNA" - , insbesondere eines Gens innerhalb eines bzw. auf einem Chromosom (s), ist. Dieses Sondenset ist **dadurch gekennzeichnet, daß** die einzige oder die Kombination der mehreren Teilsequenzen ein einmaliges Muster innerhalb des Genoms darstellen [d.h.: in der speziellen Anordnung, in der sie innerhalb der Target-DNA vorliegen, kommen sie nicht im übrigen Genom, insbesondere Chromosom vor], dass die Sonden unter Nichtanwendung eines Denaturierungsschrittes und unter Ausbildung von Watson-Crick-Bindungen zur Hybridisierung an die als zwei DNA-Einzelstränge der Target-DNA vorliegenden Teilsequenz(en) (was natürlicherweise, d.h. in vivo bzw. im nativen Zustand, wenigstens temporär bzw. vorübergehend bzw. zeitweise der Fall ist) geeignet sind, und daß die Sonden mit gleichartigen und/ oder verschiedenartigen, mikroskopisch oder spektroskopisch oder durch Magnetresonanz detektierbaren Markermolekülen gekoppelt sind, wobei im Fall der Kombination mehrerer Sondentypen und deren Kopplung mit verschiedenartigen Markermoleküle(n) jede Einheit aus Sondentyp und Markermolekül(en) praktisch das gleiche Bindungsverhalten oder den gleichen Schmelzpunkt mit dem zu ihr komplementären Einzelstrang der Target-DNA aufweist wie jede der anderen Einheiten [das heißt mit anderen Worten: alle Einheiten aus Sondentyp und Markermolekül(en) haben im wesentlichen die gleiche Bindungsenergie bzw. den gleichen Schmelzpunkt mit dem Einzelstrang des DNA-Target-Abschnitts].

Die Oligonukleotide dieses Sondensets sollten vorzugsweise 10 - 40 Nukleotide aufweisen. Entsprechendes gilt gegebenenfalls für die Oligonukleotid-äquivalenten Moleküle.

Als Markermoleküle für die erfindungsgemäßen Sondensets werden insbesondere Fluoreszenzfarbstoffe, immunhistochemische Markermoleküle, fluoreszente oder luminiszente Nanopartikel, insbesondere Nanokristalle (Quantendots), und magnetische Nanopartikel vorgeschlagen, wobei auch Kombinationen verschiedener solcher Markemolekültypen zum Einsatz kommen können.

Das erfindungsgemäße Verfahren und die erfindungsgemäßen Sondensequenzen bzw. Sondensets sind für die fokussierte Markierung von Genomregionen in der Grundlagenforschung, der angewandten Forschung und der medizinischen Diagnostik vorgesehen. Insbesondere für die medizinische Diagnostik ist ein gutes Signal-/Hintergrundsverhältnis bei der Detektion erforderlich. Dies wird mit dem erfindungsgemäßen Verfahren, insbesondere den erfindungsgemäßen Sondensequenzen und Markierungsstrategien (Nanokristalle, Fluoreszenzlöschung etc.) erreicht.

Für die mikroskopische Detektion und Abbildung der Markersignale eignen sich gleichermaßen vor allem die folgenden Mikroskopierverfahren: die Fluoreszenzmikroskopie, insbesondere optische Nahfeldmikroskopie und optische Fernfeldmikroskopie wie beispielsweise Epifluoreszenzmikroskopie, die konfokale Laser-Scanning Mikroskopie, die Wellenfeldmikroskopie, die Spatially Modulated Illumination (SMI) Mikroskopie, die 4Pi Mikroskopie und andere hochauflösende Mikroskopietechniken.

Geeignete erfindungsgemäße Sondensequenzen und Sondensets können auch mit Hilfe der Durchflußzytometrie und der Slit-Scan Flußfluorometrie sowie der Sensorik und der Magnetresonanztomographie detektiert werden.

Aufgrund dessen, daß (I) mit dem erfindungsgemäßen Verfahren kleinste Target-DNAs genau markiert werden können, und daß (II) dabei auf die Vorbehandlung des Zellmaterials, insbesondere auf eine chemische, enzymatische und/oder thermische Denaturierung, verzichtet werden kann, weil die erfindungsgemäßen Sondensequenzen und Sondensets in vivo und in situ hybridisieren (weil jede doppelsträngige DNA natürlicherweise wenigstens temporär - das heißt vorübergehend bzw. zeitweise - und abschnittsweise "offen" in Form von zwei Einzelsträngen vorliegt), ermöglicht dieses Verfahren, beispielsweise mit Hilfe der SMI-Mikroskopie, die Kompaktierung der betreffenden Genomregion abzuschätzen und so Veränderungen während der Zellfunktion oder Tumorgenese in der DNA Struktur zu erfassen.

Das erfindungsgemäße Verfahren vereinfacht in der Diagnostik den Umgang mit Patientenmaterial als Target ganz erheblich. Insbesondere brauchen physiologisch relevante Bedingungen, wie sie bei der Gewinnung des Patientenmaterials im allgemeienen vorliegen, nicht oder kaum mehr geändert zu werden. Dies ist ein entscheidender Vorteil auch in Anbetracht einer schonenden Behandlung des zu analysierenden Zellmaterials, um relevante Strukturinformationen zu konservieren.

Für die allgemeine und klinische Forschung bzw. medizinische Diagnostik eröffnet das erfindungsgemäße Verfahren und die erfindungsgemäßen Sonden insbesondere auch die vorteilhafte Möglichkeit einer spezifischen in vivo Markierung von Genomstrukturen im Zellkern für eine Analyse von Genomloci durch Magnetresonanztomographieverfahren.

Durch die Erforschung der DNA-Sequenz des menschlichen und anderer Genome stehen heute umfangreiche DNA-Sequenzbibliotheken auf entsprechenden Computern zur Verfügung. Das erfindungsgemäße Verfahren und seine Weiterentwicklung einschließlich der erfindungsgemäßen Sonden kann bei allen (diesen) Genomen mit bekannter Nukleotidsequenz für Ortsbestimmungen von Genen oder für die Detektion von Genveränderungen (Brüche, Deletionen, Mutationen etc.) eingesetzt werden. Es können somit auch spezifische Markierungen für Spezies erstellt werden, für die es bisher keine oder nur wenige DNA-Banken mit entsprechenden BAC-Klonen gibt. Dieser Vorteil ist von erheblichem, auch klinischem Interesse, z.B. im Hinblick auf eine vereinfachte Markierung von DNA-Sequenzen bestimmter Krankheitserreger.

Die Erfindung wird im folgenden anhand von Figuren und Beispielen näher erläutert.

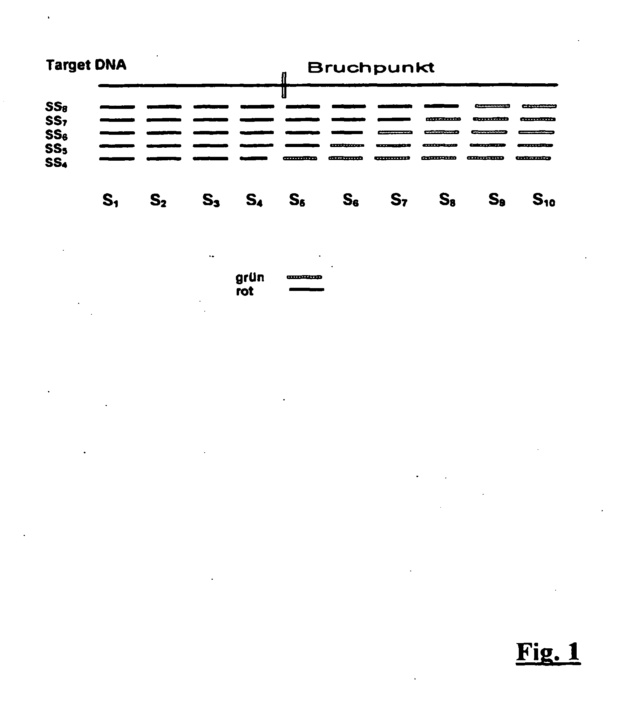

- Figur 1 zeigt:: eine schematische Darstellung der fünf Sondensetvarianten SS₄ bis SS₈ gemäß Beispiel 6, wobei von jedem Sondenset nur die ersten 10 Sonden S₁ bis S₁₀ dargestellt sind;
Sondensetvariante SS₄ enthält die Sonden S₁ bis S₄ mit Rotmarkierung (dunkler Balken) und die Sonden S₅ bis S₁₀ mit Grünmarkierung (heller Balken), Sondensetvariante SS₅ enthält die Sonden S₁ bis S₅ mit Rotmarkierung (dunkler Balken) und die Sonden S₆ bis S₁₀ mit Grünmarkierung (heller Balken), Sondensetvariante SS₆ enthält die Sonden S₁ bis S₆ mit Rotmarkierung (dunkler Balken) und die Sonden S₇ bis S₁₀ mit Grünmarkierung (heller Balken), usw;
Sondensetvariante SS₄ liefert zwei einfarbige Markersignale, denn der Bruchpunkt befindet sich in der Region zwischen der rotmarkierten (dunkler Balken) Einzelsonde S₄ und der grünmarkierten (heller Balken) Einzelsonde S₅.

### Beispiel 1: Herstellung erfindungsgemäßer Sondensets

Zur Herstellung erfindungsgemäßer Sondensets wird beispielsweise in einer DNA-Sequenzbibliothek eine zu markierende, bis auf kleinere Lücken zusammenhängende DNA-Sequenz der Gesamtlänge L, die "Target-DNA", ausgewählt. L bedeutet hierbei eine lineare geometrische Länge eines DNA-Fadens der gesamten Basensequenz (1 kbp läßt sich zu ca. L = 350 nm abschätzen). Diese Länge L kann im unbehandelten Zellkern einer zu markierenden Genomregion oder chromosomalen Subregion entsprechen, deren mittlerer Durchmesser d_{T} kleiner oder gleich der Halbwertsbreite des Hauptmaximums der effektiven Punktbildfunktion (oder auflösungsäquivalenten Größe des Detektionssystems) des für die anschließende Analyse der Target-DNA bzw. Zelle verwendeten Detektionssystems ist.

Die Target-DNA der Länge L wird nach Teilsequenzen mit geeignetem Bindungsverhalten (Bindungsenergie und/oder Bindngskinetik) und/oder geeignetem Schmelzpunkt abgesucht. Solche Teilsequenzen können beispielsweise 15mere (= Oligonukleotide, bestehend aus 15 Nukleotiden) sein, die - im einfachsten Fall - ein festes Verhältnis aus Purin- und Pyrimidinbasen aufweisen. Diese Teilsequenzen werden mit dem Rest des Genoms verglichen, und es werden nur solche ausgewählt, die in Kombination (inklusive Repetition) nicht mehr im Rest des Genoms innerhalb von beliebigen Genomabschnitten einer Länge L vorkommen. Danach werden die Teilsequenzen in entsprechende komplementäre Sondensequenzen umgeschrieben und als PNA- und/oder DNA- und/oder andere äquivalente sequenzspezifische Sonden synthetisiert. Anschließend werden die oben genannten Markermoleküle und/oder Nanokristalle ggf. über geeignete Linkermoleküle oder -molekülgruppen an diese Sonden gebunden.

Das Mischungsverhältnis der am Sondengemisch beteiligten verschiedenen Einzelsonden kann z.B. 1:1 betragen; andere Mischungsverhältnisse sind ausdrücklich zugelassen. Ferner können die Sonden dieselbe Signatur oder verschiedene Signaturen tragen.

### Beispiel 2: Markierung der Sonden(sequenzen)

Die einzelnen Sonden/Sondensequenzen können für die Detektion verschieden markiert sein:
a) Am 3' und/oder 5' Ende der Sondensequenz können Fluoreszenzmoleküle, gegebenenfalls über geeignete Linkermoleküle, angebunden sein. Diese Fluoreszenzmoleküle können für alle verschiedenen Sondensequenzen eines Sondengemischs dieselbe spektrale Signatur zeigen. In diesem Fall kann die Lokalisation und Vollständigkeit der Markierung über die Intensität detektiert werden. Die verschiedenen Sondensequenzen eines Sondensetss können aber auch verschiedene spektrale Signaturen tragen. In diesem Fall ist neben der Intensität auch die spektrale Zusammensetzung des detektierten Sondensets ein geeigneter Parameter für die Lokalisierung und Vollständigkeit der Markierung.
b) Die Fluoreszenzmoleküle können ebensogut nicht randständig in die einzelnen Sondensequenzen eingebaut werden. Für die Detektion gilt in diesem Fall dasselbe wie unter (a) beschrieben.
c) Die Fluoreszenzmoleküle können aber auch im nicht gebundenen Zustand durch Löschmoleküle in ihrer Fluoreszenz gehindert werden. Diese Moleküle können direkt an einem Ende der Sondensequenz angekoppelt sein, wie z.B. bei PNA-Sonden. Durch die Flexibilität der PNA erfolgt stets solange eine Löschung bis die spezifische Bindung der Sonde an die Target -DNA stattgefunden hat. Im Falle von DNA ist es möglich, die Fluoreszenz durch zusätzliche Nukleotide oder Moleküle in einer Stamm-Schleifen-Struktur der Sondensequenz (sog. Smart-Probes oder Hairpin-Probes) zu unterdrücken. Erst wenn die Schleife spezifisch bindet, wird der Stamm geöffnet und die Fluoreszenz leuchtet.
d) Anstelle von Fluoreszenzmolekülen können magnetische Markermoleküle, Markermoleküle mit einer hohen Affinität zu Farbstoffkomplexen, z.B. Steroide oder Haptene, oder Moleküle einer hohen atomaren Wechselwirkung zu Rasterkraftmikroskopiespitzen in die Sondensequenzen eingebaut bzw. mit diesen gekoppelt werden. Im Falle der Markermoleküle mit einer hohen Affinität zu Farbstoffkomplexen gelten die Ausführungen in (a), (b) und (c) bezüglich der anzubindenden Farbstoffkomplexe analog.
e) Die Markierung kann ebensogut mittels fluoreszierender/luminiszierender und/oder magnetischer Nanopartikel (Quantendots) erfolgen.

Grundsätzlich werden alle Sonden derart markiert, dass ihr Markersignal im Fall der Bindung an die Target-DNA signifikant gegenüber einer unspezifischen Hintergrundmarkierung hervortritt.

### Beispiel 3: Ortsbestimmung eines Gens X im Genom G

Gen X ist die Target-DNA "T". Für diese Target-DNA wird ein Anfangs- und Endnukleotid festgelegt, um im folgenden die dazwischen liegende Region spezifisch zu markieren.

In einer Genomdatenbank wird eine Datenbankrecherche durchgeführt, und es wird mindestens eine DNA-Sequenz, vorzugsweise eine Kombination von DNA-Sequenzen bestimmt - die sogenannten "Teilsequenzen" -, die innerhalb der Target-DNA (d.h.: in der Region bzw. über die Länge L der Target-DNA ) kolokalisieren, nämlich gemeinsam auftreten, und die in dieser Kombination nicht auch im übrigen Genom G in einem beliebigen Genomabschnitt einer Länge L vorkommen.

Übereinstimmend mit oder komplementär zu diesen Teilsequenzen werden einzelsträngige Sondensequenzen hergestellt, die über Watson-Crick-Bindungen mit dem passenden DNA-Einzelstrang dieser Teilsequenzen hybridisieren können - sobald diese Teilsequenz als zwei Einzelstränge vorliegt, was von Natur aus von Zeit zu Zeit (= temporär, vorübergehend) immer wieder vorkommt.

Die Sondensequenzen bzw. Teilsequenzen werden dabei nach folgenden Kriterien ausgewählt:
- Es müssen so viele Sondensequenzen innerhalb (in der Region bzw. über die Länge) der Target-DNA binden, dass innerhalb der Auflösungsgrenzen des zur Anwendung vorgesehenen detektierenden Systems mindestens eine Sonde liegt.
- Wenn die Target-DNA mit einer einzigen Sondensequenz definiert werden kann, weil die dazugehörige (komplementäre) Teilsequenz im betreffenden Genom einzigartig ist, genügt die Bereitstellung dieser einzigen Sondensequenz.
- Kann die Target-DNA nicht mit einer einzigen Teilsequenz und damit SondenSequenz definiert werden, ist die Bereitstellung einer Kombination von Sondensequenzen, im folgenden auch "Sondengemisch" genannt, erforderlich. Die Sondensequenzen dieser Kombination bzw. dieses Sondensets werden jeweils so ausgewählt, dass die Einzelsonden alle gemeinsam nur in der Region der Target-DNA kolokalisieren (= gemeinsam auftreten), nicht auch im übrigen Genom.
- Um eine einheitliche Bindung der Einzelsonden eines Sondensets an die Target-DNA zu gewähren, sollten die verschiedenen Sondensequenzen so ausgewählt werden, dass sie dasselbe Bindungsverhalten oder denselben Schmelzpunkt mit dem DNA-Strang aufweisen. Diese Parameter können auch in vorgegebenen Grenzen variiert werden.

Die gewählten Sondesequenzen werden anschließend mit Markermolekülen gekoppelt. Besonders geeignete Markierungen sind die folgenden:
(a) Die Sonden werden mit Fluoreszenzfarbstoffen gekoppelt, deren Intensität und/oder spektrale Signatur (Farben und/oder Fluoreszenzlebensdauer) als Detektionsparameter dient.
(b) Die Sonden werden mit fluoreszenten/luminiszenten Nanokristallen (Quantendots) gekoppelt.
(c) Es werden Sondenkonstrukte erzeugt, die durch Fluoreszenzlöschmoleküle oder aufgrund der Anordnung der Fluorochrome in der Sonden(nukleotid)sequenz nur dann fluoreszieren, wenn sie gebunden sind, so dass der Hintergrund durch nicht gebundene Sonden in Zellen unterdrückt wird.
(d) Die Sonden werden mit nicht-fluoreszenten / nicht-optischen Markermolekülen gekoppelt, die den Nachweis der Markierung mittels nicht-optischer Detektionsverfahren wie z.B. atomare Kraftmikroskopie oder Magnetresonanz ermöglichen.
(e) Die Sonden werden mit Molekülen gekoppelt, die eine anschließende Markierung wie unter (a) bis (d) beschrieben ermöglicht.
(f) Die Sonden werden so konstruiert, dass sie aufgrund ihrer chemischen Eigenschaften eine geeignete Eigenfluoreszenz aufweisen oder aufgrund anderer spezifischer Eigenschaften auch ohne Modifikation detektiert werden können.

Das Sondegemisch wird per Mikroinjektion in die das Genom G enthaltende Zelle injiziert. Eben so gut kann auch jedes andere bekannte Verfahren zum Einbringen von Oligonukleotiden in lebende Zellen eingesetzt werden.

Nach einer Inkubationszeit, deren Dauer sich bei lebenden, proliferierenden Zellen nach dem Fachmann bekannten Zellzykluszeiten bemisst, wird die betreffende Zelle mit Hilfe des Detektionssystems, das für die gewählten Markermoleküle geeignet ist, -im Fall von Fluoreszenzfarbstoffen folglich mit Hilfe eines Fluoresszenzmikroskops - untersucht und die Markersignale detektiert:
Die Lokalisierung der Target-DNA "T" der Länge L kann beispielsweise dadurch erfolgen, dass a) alle Sonden für die gegebenenfalls mehreren verschiedenen Teilsequenzen mit Fluorochromen derselben spektralen Signatur S₁ markiert sind; oder dass b) eine Gruppe von Sonden mit einer spektralen Signatur S₁ und eine andere Gruppe mit einer spektralen Signatur S₂, bzw. weitere Teile mit spektralen Signaturen Sₙ markiert werden; oder dass c) eine Kombination von a) und b) durchgeführt wird.

In Fall a) erfolgt die Diskriminierung (Unterscheidung) der an die Target-DNA "T" gebundenen Sonden von unspezifisch gebundenen Sonden anhand der erhöhten Intensität des Fluoreszenzsignals: Da vorausgesetzt ist, dass der Durchmesser d_{T} der Target-DNA kleiner ist als die Halbwertsbreite des Hauptmaximums der effektiven Punktbildfunktion FWHM (oder auflösungsäquivalenten Größe des Detektionssystems), addieren sich die Fluoreszenzemissionintensitäten der spezifisch gebundenen Sonden im Bereich der Target-DNA zu einer Gesamtintensität, während die nicht spezifisch gebundenen Sonden zufällig räumlich im Objekt verteilt liegen, wobei ihr mittlerer Abstand bei geeigneten Bedingungen größer ist als die FWHM. Als Konsequenz ist die Intensität dieser vereinzelten Fluoreszenzsignale ("Hintergrund") erheblich geringer. Sind beispielsweise 10 Sonden spezifisch an die Genomregion T gebunden und die übrigen Sonden im Präparat zufällig verteilt, dann kann der Ort der Genomregion T aufgrund der ca. 10mal so starken Intensität seines Fluoreszenzsignals identifiziert werden.

In Fall b) erfolgt die. Identifizierung der spezifisch and die Target-DNA "T" gebundenen Sonden anhand der Kolokalisation von Fluoreszenzsignalen unterschiedlicher spektraler Signatur. Beispielsweise enthält T lediglich 3 Bindungsstellen für Sonden t₁, t₂, t₃, die jeweils mit den spektralen Signaturen S₁, S₂ und S₃ markiert wurden. In diesem Falle wird sich die Intensität der von den einzelnen Sonden t₁, t₂ und t₃ detektierten Fluoreszenzsignale am Ort T nicht von der Intensität der "Hintergrundssignale" unterscheiden. Der Ort T ist jedoch charakterisiert durch das gleichzeitige Auftreten von Fluoreszenzsignalen mit den spektralen Signaturen S₁, S₂ und S₃ (gegebenenfalls nach Korrektur einer auftretenden chromatischen Verschiebungen = spektrale Kolokalisation).

Fall c) ist eine Kombination der beiden Ausführungsformen a) und b): Die Detektion des Ortes von T erfolgt anhand eines im Vergleich zum Hintergrund stärkeren Fluoreszenzsignals von Fluorochromen einer bestimmten spektralen Signatur und zusätzlich anhand der spektralen Kolokalisation von zwei oder mehr spektralen Signaturen.

Bei gleichzeitiger Abbildung von Markersignalen und Genom zeigt das Vorhandensein, die Intensität und gegebenenfalls das gleichzeitige Auftreten der verschiedenen Markersignale an einem bestimmten Ort an, wo die Target-DNA bzw. das Gen X in diesem Genom lokalisiert ist.

### Beispiel 4: Ortsbestimmung mehrerer verschiedener Gene in demselben Genom

Zwei verschiedene Gene, das heißt zwei verschiedene Target-DNAs T₁, T₂ mit den jeweiligen Längen L₁, L₂ können wie folgt unterschieden werden:
a) Die für T₁ spezifischen Sondensequenzen werden alle mit derselben spektralen Signatur S₁ markiert; die für T₂ spezifischen Sonden werden alle mit einer spektralen Signatur S₂ markiert. Der Ort von T₁ wird aufgrund des stärkeren (erhöhten) Fluoreszenzsignals der spektralen Signatur S₁ detektiert; der Ort von T₂ wird aufgrund des stärkeren (erhöhten) Fluoreszenzsignals der spektralen Signatur S₂ detektiert.
b) Die für T₁ spezifischen Sonden werden mit Fluorochromen der spektralen Signaturen S₁, S₂, S₃ markiert; die für T₂ spezifischen Sondens werden mit Fluorochromen der spektralen Signaturen S₄, S₅, S₆ markiert. Der Ort von T₁ wird in diesem Falle durch die spektrale Kolokalisation von S₁, S₂, S₃ detektiert; der Ort von T₂ wird durch die spektrale Kolokalisation von S₄, S₅, S₆ detektiert.
c) Die Vorgehensweisen a) und b) werden miteinander kombiniert, wobei die Anzahl und Kombination der spektralen Signaturen geeignet variiert werden kann. Es versteht sich für den Fachmann von selbst, daß diese Vorgehensweisen a) bis c) analog auch bei mehr als 2 Target-DNAs z.B. mit Hilfe einer entsprechenden Erweiterung der Anzahl der Signaturen durchgeführt werden können.

### Beispiel 5: Ortsbestimmung des Gens FMR1 auf dem humanen Chromosom X

Veränderungen am Gen FMR1 führen zum so genannten Fragilen X-Syndrom, das eine der häufigsten Ursachen erblicher kognitiver Behinderung darstellt.
Die Nukleotidsequenz des Gens FMR1 ist im Stand der Technik bekannt und beispielsweise über eine Datenbank (z.B. NCBI, Ensembl, u.a.) dem Fachmann zugänglich. Diese Nukleotidsequenz des Gens FMR1 ist im vorliegenden Ausführungsbeispiel die Target-DNA.
Diese Target-DNA wurde hinsichtlich solcher Teilsequenzen analysiert, die ein einmaliges Muster innerhalb des FMR1-Gens darstellen. Zu den gefundenen Teilsequenzen wurden die in Tabelle 1 aufgelisteten einzelsträngigen Sondensequenzen bereitgestellt, die mit den Teilsequenzen übereinstimmen bzw. komplementär dazu sind.

Alle diese Sondensequenzen hybridisieren über eine Watson-Crick-Bindung mit dem einen oder anderen DNA-Einzelstrang der Target-DNA, sobald dieser DNA-Abschnitt des X-Chromosoms in Form von zwei Einzelsträngen vorliegt - was in vivo naturgemäß wenigsten von Zeit zu Zeit passiert, beispielsweise während der Zellteilung und dabei insbesondere während der DNA-Verdopplung.

Für die Durchführung der Ortsbestimmung des FMR1-Gens kann der Fachmann bei Bedarf die Zellteilung der zu untersuchenden Patientenzelle stimulieren bzw. induzieren und dadurch die natürliche temporäre Aufspaltung der DNA in zwei DNA-Einzelstränge herbeiführen.

Zwei oder mehr dieser Sondensequenzen, die ein Purin:Pyrimidin-Verhältnis oder Pyrimidin:Purin-Verhältnis von mindestens 3:16 aufweisen, werden zu einem Sonden-Set kombiniert. Die Zahl der verwendeten Einzelsonden hängt dabei von der Nachweisempfindlichkeit des verwendeten Mikroskops ab. Bei einem Mikroskop mit geringer Empfindlichkeit müssen mehr Sonden verwendet werden als bei einem Mikroskop mit hoher Empfindlichkeit, da die Einzelsonden typischerweise nur ein oder zwei Fluorochrome tragen.
Prinzipiell können auch andere Sondensets für diese Genomregionen erstellt werden, die gemäß anderer erfindungsgemäßer Parameter definiert sind.

Die Sondensequenzen des Sondensets werden mit Fluoreszenzmolekülen (z.B. Oregon Green) gleicher Farbsignatur gekoppelt, die mit Laserlichtquellen (z.B. bei 488 nm) angeregt werden, und deren Fluoreszenz mikroskopisch über eine CCD-Kamera detektiert wird. Jede Einheit aus Sondensequenz und Markermolekül(en) weist praktisch das gleiche Bindungsverhalten und unter den gegebenen Experimentierbedingungen einen annähernd gleichen Schmelzpunkt mit dem zu ihr komplementären Einzelstrang der Target-DNA auf wie jede der anderen Einheiten.

Die Sondensequenzen werden in vitro mittels Mikroinjektion in die Zelle einer Patientenprobe eingebracht und bekannten Hybridisierungsbedingungen ausgesetzt, beispielsweise 26 Stunden Inkubation bei 37°C unter Standard-Zellkultur-Bedingungen. Nach Ablauf der Hybidiserungsphase werden die Zellen fixiert und die Markersignale in den Zellkernen detektiert. Anhand des radialen Abstandes zum Zellmittelpunkt und durch Vergleich mit Daten aus Referenzexperimenten mit fixierten Zellen und X-chromosomalen Standard-FISH-Sonden (FISH= Fluoreszenz-In-Situ-Hybrisisierung) wird der Ort der Target-DNA auf dem X-Chromosom identifiziert. Ergänzend kann mittels SMI-Mikroskopie (SMI= Spatially Modulated Illumination) die DNA-Kompaktierung gemessen werden und aus diesen Messergebnissen auf den genetischen Zustand der Markierungsregion geschlossen werden.

### Beispiel 6: Einsatz des erfindungsgemäßen Verfahrens in der medizinischen Diagnostik

Das Gen " RyR2" auf Chromosomen 1 des menschlichen Genoms ist mit zwei Krankheiten assoziiert, bei denen es zu einer balancierten Translokation zwischen Chromosom 1 und Chromosom 14 kommt, nämlich (a) der Katecholaminergen polymorphen ventrikulären Tachykardie und (b) der Rechtsventrikulären Dysplasie Typ 2.
Der Bruchpunkt für diese Translokation liegt innerhalb des RyR2-Gens. Er muß jedoch bei den Patienten genau bestimmt werden, da seine Lage individuell verschieden sein kann und deshalb bei der Translokation eine Transkription des defekten Gens gestartet werden kann oder nicht.
Um die Lage des Bruchpunktes auf dem Gen eingrenzen zu können, wird zunächst das Gen RyR2, das hier die Target-DNA darstellt, anhand von bekannten Genomdatenbanken hinsichtlich mehrerer Teilsequenzen analysiert, die ein Purin:Pyrimidin-Verhältnis von 0:N (mit N=14, 15, ... bis 21) besitzen. Anschließend wird ein Sondenset von 16 Einzelsonden bereitgestellt, die mit den Teilsequenzen der Target-DNA übereinstimmen bzw. komplementär dazu sind. Alle 16 Sonden hybridisieren über Watson-Crick-Bindung mit komplementären Sequenzen entlang des RyR2-Gens, wobei nach erfolgter Hybridisierung die Einzelsonden über die gesamte Länge des Gens verteilt vorliegen. Die Zusammensetzung dieses Sondensets ist in Tabelle 2 dargestellt.
Die Sondensequenzen werden an jedem ihrer Enden mit Fluoreszenzmolekülen gleicher Farbsignatur gekoppelt. Dabei werden für jede Sondensequenz zwei Aliquots hergestellt, die sich in ihrer Farbsignatur unterscheiden, z.B. Oregon green (grün) und TAMRA (rot).
Zur Bestimmung der Lage des Bruchpunktes werden 16 Varianten des Sondensets bereitgestellt, die sich in der Farbmarkierung der Sonden derart unterscheiden, dass pro Variante der Anteil rotmarkierter Sonden um eine Sonde zunimmt und der Anteil grünmarkierter Sonden um eine Sonde abnimmt. Dabei erfolgt die Markierung der Sonden mit zunächst rot und später grün in der Reihenfolge ihrer Anordnung auf der Target-DNA. Es resultieren 16 Sondensetvarianten SS₁ bis SS₁₆, wobei die Sondensetvariante SS₁ die Sonde S₁ mit Grünmarkierung und die Sonden S₂ bis S₁₆ mit Rotmarkierung enthält, die Sondensetvariante SS₂ enthält die Sonden S₁ und S₂ mit Grünmarkierung und die Sonden S₃ bis S₁₆ mit Rotmarkierung, die Sondensetvariante SS₃ enthält die Sonden S₁, S₂ und S₃ mit Grünmarkierung und die Sonden S₄ bis S₁₆ mit Rotmarkierung usw. (siehe Fig. 1).
Mit jeder dieser 16 Sondenset-Varianten wird je ein Hybridisierungsdurchgang mit der Target-DNA durchgeführt. Hierfür werden die Sondensets auf fixierte Zellen einer Patientenprobe appliziert und Standard-Hybridisierungsbedingungen ausgesetzt.
Nach Ablauf der Hybridisierungsphase werden die Markersignale in den Zellkernen detektiert.
Diejenige Sondensetvariante, die zwei einfarbige Markersignale liefert, zeigt die Lage des Bruchpunktes an, denn der Bruchpunkt befindet sich in der Region zwischen den beiden Sondenregionen Sₙ und Sₙ₊₁ der Sondensetvariante SSₙ, die nach der Hybridisierung ein einfarbiges Markersignal (grün oder rot) liefert (siehe Fig. 1).
Um die Lage des Bruchpunktes noch genauer zu bestimmen, wird für die Region zwischen den beiden ermittelten Sondenregionen Sₙ und Sₙ₊₁ ein neues Sondenset SS^{*} erstellt, dessen Einzelsonden ein Purin-Pyrimidinverhältnis von 1:N (mit N=14, 15, ... bis 21) aufweisen.
Von diesem Sondenset SS* werden wieder Varianten SS*₁ bis SS*ₙ bereitgestellt, die sich analog der Sondensetvarianten SS₁ bis SS₁₆ in der Farbmarkierung (beispielsweise rot oder grün) ihrer Einzelsonden unterscheiden.
Mit jeder dieser n Sondenset-Varianten wird wieder je ein Hybridisierungsdurchgang mit der Target-DNA durchgeführt, wobei die Sondensets wieder auf fixierte Zellen einer Patientenprobe appliziert und Standard-Hybridisierungsbedingungen ausgesetzt werden.
Nach Ablauf der Hybridisierungsphase werden wiederum die Markersignale in den Zellkernen detektiert.
Diejenige Sondensetvariante, die zwei einfarbige Markersignale liefert, zeigt wieder an, zwischen welchen Sondenregionen S*ₙ und S*ₙ₊₁ der Sondensetvariante SS*ₙ der Bruchpunkt lokalisiert ist.

### Beispiel 7: Einsatz des erfindungsgemäßen Verfahrens in der Pathologie

Die erhöhte Anzahl an Kopien der Gene "Her2neu" und "GRB7" auf Chromosm 17 des menschlichen Genoms ist für einen Therapieentscheid beim duktalen Mammakarzinom von wesentlicher Bedeutung. Die beiden Gene haben eine Länge von ca. 30 kb (Her2neu) und ca. 10 kb (GRB7) und liegen nur etwa 10 kb voneinander entfernt.
Herkömmliche, allgemein zugängliche Sonden aus modifizierten BAC-Klonen können daher diese Gene nicht separat darstellen.
Erfindungsgemäß wird für die mikroskopische Diagnostik an Gewebeschnitten für jedes dieser beiden Gene ein Sondenset aus je 18 Einzelsonden erstellt, wobei die Gesamtzahl der Nukleotide aller Einzelsonden pro Sondenset 300 oder mehr und der Unterschied in der Nukleotidgesamtzahl zwischen den beiden Sondensets höchstens 5 % betragen sollte.
Ein konkretes Beispiel für eine gut geeignete Zusammensetzung dieser beiden Sondensets ist in Tabelle 3 bzw. Tabelle 4 dargestellt.
Für die mikroskopische Analyse werden die Einzelsonden eines jeden Sets mit je zwei Fluoreszenzmolekülen gleicher Farbsignatur markiert. Die beiden Sondensets erhalten verschieden Farbsignaturen. Die Hybridisierung erfolgt gemäß Standard-Protokollen, da die Sonden Watson-Crick-Bindungen eingehen können.
Die mikroskopische Analyse erfolgt anhand von Bildaufnahmen mittels einer CCD-Kamera in einem Epifluoreszenzmikroskop. Letztendlich wird die Anzahl der Markierungen (Farbpunkte) ausgezählt und daraus auf die Anzahl Kopien des betreffenden Gens geschlossen. Die gefundene Anzahl liefert dem Mediziner den Hinweis auf die geeignete Therapie.

### Beispiel 8: Weitere Einsatzmöglichkeiten

Das erfindungsgemäße Verfahren kann auch mit beliebig fixiertem Untersuchungsmaterial durchgeführt werden. Damit gehen insbesondere die Vorteile einher, daß bei geeigneter Sondenwahl ein Denaturierungsschritt entfallen kann, und dass das Sondengemisch genauso einfach gehandhabt werden kann wie z.B. etablierte DNA-Färbungen in der klinischen Zytogenetik:
Das Sondengemisch wird auf das Präparat gegeben, inkubiert, evtl kurz gewaschen und schließlich mikroskopisch ausgewertet.
Ein weiterer Vorteil gegenüber bekannten FISH-Verfahren bei Raumtemperatur besteht darin, dass keine toxisch wirkenden chaotropen chemischen Agenzien eingesetzt werden müssen, die bei der Handhabung allergische Reaktionen hervorrufen können.

In der Sensorik können entsprechende DNA-Chips konstruiert werden, die Target-DNAs bei Anlagerung an gegebene Sondensequenzen erkennen, ohne dass der Chip oder das Untersuchungsmaterial zusätzlich einer chemischen und/oder thermischen Behandlung unterworfen werden muss. Auch die Anforderungen an das zur Auswertung dieser DNA-Chips verwendete optische System können erheblich reduziert werden. Zum Beispiel ist es möglich, Mikroskopoptiken von geringerer numerischer Apertur einzusetzen, sofern die Dimensionen des Chips entsprechend angepasst und beispielsweise Nanokristalle verwendet werden. Da heute zur optischen Analyse von DNA-Chips sehr komplexe Systeme zur Verfügung stehen, kann hierdurch das Verfahren wesentlich ökonomischer gestaltet werden.

Das erfindungsgemäße Verfahren hat sich in der Praxis an folgenden Genen als sehr gut anwendbar erwiesen:
NRAS[1], AKT3[1], CDC2L1(p58)[1], CDC2L2(p58)[1], ABL2[1], RYR2[1], MSH2[2], GNLY[2], RASSF1[3], FHIT[3], EGF[4], ENC1[5], MCC[5], HMNR[5], ECG2[5], PIM1[6], ABCB1 (MDR)[7], MET[7], (C-)MYC[8], CDKN2A[9], ABL1[9] PTEN[10], PGR[11], ATM[11], KRAS2[12], RB1[13], PNN[14], IGH[14], SNRPN[15], IGF1R[15], UBE3A[15], PML[15], FANCA[16], CDH13[16], D17S125[17], ERBB2 (HER2neu)[17], TP53 (p53)[17], PSMD3(p58subunit)[17], RARA[17], GRB7[17], LAMA3[18], AKT2[19], TNFRSF6B[20], MYBL2[20], PTPN1[20], ZNF217[20], PCNT2[21], TFF1-3[21], PDGFB (SIS)[22], TBX1[22], BCR[22], ERAS[X], PIM2[X], RAB9A[X], DAZ4[Y], DAZ3[Y].

**Tabelle 1: Einzelsonden des Sondensets in Beispiel 5 (für die Ortsbestimmung des Gens FMR1 des humanen X-Chromosoms)**

| | |
|---|---|
| (1) | AACCTTTCTTTTCTCTTCCAA |
| (2) | CCAAAAGAAGAAAGAAGGTC |
| (3) | TTAAAGAGAGGAGAAGGTG |
| (4) | GTGAGAGAGAAAGAGAAAGAGAGTG |
| (5) | TATTCTTTCCTTTTCTTTTAC |
| (6) | TTGAGAGGGAAAGGGAATA |
| (7) | ATGGGAGAGGAGGAGAAGATG |
| (8) | AGTTCCTTTTCCTTTCCAT |
| (9) | GATCCTTCCTTTCTCCCCTGT |
| (10) | CTGAAGGAAGGAAGGAGGGAGGAAGGAAGGAAGCA |
| (11) | GTAGAGGGAAGGGAGAAAGGTG |
| (12) | TCAAAAGAAGGAGAAGATG |
| (13) | TGTCTTCCTTTCTTCCTCCAT |
| (14) | TGTCTCTCTCTCTCCTCCCCCCC |
| (15) | CACCCCTCTCTCTCCTTCTCTCTTTTCTGT |
| (16) | TGCTCCCCTCCCTCCTCAC |

**Tabelle 2: Einzelsonden des Sondensets SS in Beispiel 6 (für die Bruchpunkt-Lokalisierung auf dem Gen RyR2 des humanen Chromosoms 1)**

| | |
|---|---|
| (1) | CCTCCTCTTCTCTCCCTCCC |
| (2) | TCTTCCTTTTCTCTCT |
| (3) | CTTTCCCTCCCTCTT |
| (4) | CCTTCTTCTTTCCCC |
| (5) | CCTCCTCTTTTCTCCT |
| (6) | TTCCTTCCTTCCCTCTTTCC |
| (7) | CTCTTTTCTTTTCTTTTCTTTCTT |
| (8) | CTCCTTCCTTTCTCTC |
| (9) | TTCTCTTTCTTCTCTTCT |
| (10) | TTTCTCTTTCCTCCT |
| (11) | TCTTTCCCTTTCCCTCC |
| (12) | CCTCCCTTCCCCCTTCC |
| (13) | CTCCTTTTCCCTTCCCT |
| (14) | CCCTTTCCCTTTCTCTCCC |
| (15) | CCCCCTTCCTCCCCTTTC |
| (16) | TCTCTCTCCCTCCCCTC |
| (17) | TCTTCTCTCCTCTTCTCTCCTC |

**Tabelle 3: Einzelsonden des Sondensets für GRB 7 in Beispiel 7**

| | |
|---|---|
| (1) | CTTCCTCCCTTCTCCTCC |
| (2) | CCCTCCCCCCTCCCTCCC |
| (3) | CCCCTTCCTCCTCCCT |
| (4) | CCTCCTTCTCCCCTCT |
| (5) | CCTCTCTCCCTTTTTCTTCTT |
| (6) | AGAAGGGAAGGGAGGGA |
| (7) | AGAGGGGAAGGGAGGAGG |
| (8) | CTCTCTTTCTCTCCCC |
| (9) | CCTCTCTCCTCTCCTTCC |
| (10) | AGGAGGGAGGAAGAGAGGG |
| (11) | CCCCCTCTCCTTCTCCT |
| (12) | GGGGAGGAGAGAAAAGAAGGAG |
| (13) | CCTCCCTTTCCTCTCCC |
| (14) | AGGAGAGGAGAGAGAGGGAAGA |
| (15) | CCTTCCTTCCCCCCTCTCCCCT |
| (16) | GGGGGAGGGGAAGAAGGAGGG |
| (17) | TTCCTTTCTCCTCCTC |
| (18) | CTCCCTTTTCTCTTCTT |

**Tabelle 4: Einzelsonden des Sondensets für Her2neu in Beispiel 7**

| | |
|---|---|
| (1) | CTTTCTCCCCTTCTCCCTC |
| (2) | AAGGAGAAAAGGAGGA |
| (3) | GAGGGGAGAAGGGAGG |
| (4) | CCTCCTCTCTCTCCC |
| (5) | GGGAAGGAGAAGAGGAAGG |
| (6) | CCCCTCCTCCTTCCTCT |
| (7) | AGAGGAAGAGAAGAA |
| (8) | CCTTCTTTCCTCTCTCCTTCCC |
| (9) | CCCTTTCTCCTCCCCC |
| (10) | TCTCTTTTCCTTTCTCTTCCCCCTCCTC |
| (11) | AAAGGAAGAGAAGAA |
| (12) | CCCCTTTCCCTTCCCT |
| (13) | CCCCCCTTCCTCTCCTCTT |
| (14) | GGGGGGGAGGGAAGAGAGAAAGAGA |
| (15) | GAAGAGAGGGAGAAAG |
| (16) | GAGAAGGAAGGAGAGAG |
| (17) | CCTCTTTCCTCCTCTC |
| (18) | TCCTTCCCTCCCCCTCT |

## Patentansprüche

1. FISH (fluorescence-in-situ-hybridization)-Verfahren zur mikroskopischen Analyse der Lokalisation eines ausgewählten intrazellulären, nativen Genomabschnitts mit bekannter Nukleotidsequenz - im folgenden "Target-DNA" - , innerhalb eines Genoms in situ, **dadurch gekennzeichnet,**
(1.) **dass** die Target-DNA anhand von Genomdatenbanken hinsichtlich
(a) einer einzigen oder (b) mehrerer gleichartiger oder verschiedener Teilsequenzen analysiert wird, und solche Teilsequenzen ausgewählt werden, die alleine oder in Kombination ein einmaliges Muster innerhalb des Genoms darstellen, welches im Rest des Genoms außerhalb der Target-DNA nicht mehr innerhalb von beliebigen Genomabschnitten mit der Länge der Target-DNA vorkommen,
- wobei jede Teilsequenz eine Länge von 8 bis 40 Nukleotiden aufweist,
(2.) **dass** diese Teilsequenzen in entsprechende komplementäre Sondensequenzen umgeschrieben und als einzelsträngige Sondensequenzen bereitgestellt werden,
- die mit der/den Teilsequenz(en) der Target-DNA übereinstimmen oder komplementär dazu sind,
- und die über eine Watson-Crick-Bindung zur Hybridisierung an die natürlicherweise wenigstens temporär als zwei DNA-Einzelstränge der Target-DNA vorliegenden Teilsequenz(en) geeignet sind,
(3.) **dass** diese Sondensequenzen mit gleichartigen und/ oder verschiedenartigen, mikroskopisch oder spektroskopisch oder durch Magnetresonanz detektierbaren Markermolekülen gekoppelt sind,
wobei im Fall (1b) einer Kombination mehrerer Teilsequenzen bzw. Sondensequenzen und deren Kopplung mit verschiedenen Markermoleküle(n) jede Einheit aus Sondensequenz und Markermolekül(en) praktisch das gleiche Bindungsverhalten oder den gleichen Schmelzpunkt mit dem zu ihr komplementären Einzelstrang der Target-DNA aufweist wie jede der anderen Einheiten,
(4.) **dass** diese Sondensequenzen mittels bekannter Verfahren unter Nichtanwendung eines Denaturierungsschrittes derart in die Zelle eingebracht und mit der Target-DNA zusammengebracht werden, daas sie mit den entsprechenden, temporär als zwei DNA-Einzelstränge der Target-DNA vorliegenden Teilsequenz(en) der Target-DNA unter Ausbildung von Watson-Crick-Basenpaarung hybridisieren,
(5.) **dass** die emittierten Markersignale detektiert werden, und
(6.) **dass** anhand des Vorhandenseins und/oder der Intensität und/oder desgleichzeitigen Auftretens von verschiedenen Markersignalen der Ort der Target-DNA auf dem Genom identifiziert wird.

2. Verfahren nach Anspruch 1, **dadurch gekennzeichnet, dass** es in vivo durchgeführt wird.

3. Verfahren nach Anspruch 1 oder 2, **dadurch gekennzeichnet, dass** jede Teilsequenz 10 bis 40 Nukleotide aufweist.

4. Verfahren nach einem der Ansprüche 1 bis 3, **dadurch gekennzeichnet, dass** die Markermoleküle Fluoreszenzfarbstoffe und/oder immunhistochemische Markermoleküle, und/oder fluoreszente oder luminiszente oder magnetische Nanopartikel, insbesondere Nanokristalle (Quantendots) sind.

5. Verfahren nach einem der Ansprüche 1 bis 4 zur Ortsbestimmung und zum Nachweis von Punktmutationen und/oder Genom-Bruchpunkten und/oder äquivalenten Sequenzdefekten, **dadurch gekennzeichnet,**
**dass** in Schritt (1) die Target-DNA hinsichtlich mehrerer, gleichartiger oder verschiedener Teilsequenzen t₁-tₙ analysiert wird,
**dass** in Schritt (3) die Sondensequenzen S₁-Sₙ mit verschiedenartigen, mikroskopisch oder spektroskopisch oder durch Magnetresonanz detektierbaren Markermolekülen gekoppelt sind, und
**dass** in Schritt (6) die Abwesenheit der Markersignale einer oder mehrerer Sondensequenzen Sₓ bei gleichzeitiger Anwesenheit der Markersignale der Sondensequenzen Sₙ-Sₓ das Vorliegen einer Punktmutation oder eines Genombruchpunktes oder eines äquivalenten Sequenzdefektes anzeigt.

6. Verfahren nach einem der Ansprüche 1 bis 5, **dadurch gekennzeichnet, dass** die Sondensequenzen mittels Mikroinjektion in die Zelle eingebracht werden.

7. Verwendung eines Sondensets mit den Merkmalen:
- es enthält einen einzigen oder mehrere Sondentyp(en) S₁ - Sₙ,
- - wobei jeder Sondentyp Sᵢ aus einem Oligonukleotid besteht,
- - - das eine Länge von 8 bis 40 Nukleotiden aufweist, und
- - - das komplementär zu oder identisch mit einer einsträngigen Teilsequenz tᵢ eines intrazellulären, doppelsträngigen Genomabschnitts mit bekannter Nukleotidsequenz - im folgenden "Target-DNA" - ist, wobei
die einzige Teilsequenz tᵢ oder die Kombination der mehreren Teilsequenzen t₁-tₙ stellt ein einmaliges Muster innerhalb des Genoms darstellt und im Rest des Genoms außerhalb der Target-DNA nicht mehr innerhalb von beliebigen Genomabschnitten mit der Länge der Target-DNA vorkommt,
- die Sonden sind unter Nichtanwendung eines Denaturierungsschrittes zur Hybridisierung an die natürlicherweise wenigstens temporär als zwei DNA-Einzelstränge der Target-DNA vorliegenden Teilsequenz(en) unter Ausbildung von Watson-Crick-Bindungen geeignet,
- und die Sonden sind mit gleichartigen und/ oder verschiedenartigen, mikroskopisch oder spektroskopisch oder durch Magnetresonanz detektierbaren Markermolekülen gekoppelt,
wobei im Fall der Kombination mehrerer Sondentypen und deren Kopplung mit verschiedenartigen Markermoleküle(n) jede Einheit aus Sondentyp und Markermolekül(en) praktisch das gleiche Bindungsverhalten oder den gleichen Schmetzpunkt mit dem zu ihr komplementären Einzelstrang der Target-DNA aufweist wie jede der anderen Einheiten; in einem FISH (fluorescence-in-situ-hybridization)-Verfahren nach Anspruch 1.

8. Verwendung nach Anspruch 7, **dadurch gekennzeichnet, dass** das Oligonukleotid 10-40 Nukleotide aufweist.

9. Verwendung nach Anspruch 7 oder 8, **dadurch gekennzeichnet, dass** die Markermoleküle Fluoreszenzfarbstoffe und/oder immunhistochemische Markermoleküle, und/oder fluoreszente oder luminiszente oder magnetische Nanopartikel, insbesondere Nanokristalle (Quantendots) sind.

## Claims

1. FISH (fluorescence-in-situ-hybridization)-method for the microscopic analysis of the localization of a selected intracellular native genome segment having a known nucleotide sequence - "target DNA" hereinafter - within a genome in situ, **characterized**
(1.) **in that** the target DNA is analyzed on the basis of genome databases in relation to (a) a single or (b) a plurality of the same or different partial sequences and those partial sequences are selected, which alone or in combination represent a unique pattern within the genome, said pattern does not occure outside the target DNA in the remainder of the genome within any genom-segment having the length of the target DNA,
- wherein each partial sequence has a length of 8 to 40 nucleotides,
(2.) **in that** these partial sequences are transcribed into corresponding complementary probe sequences and provided as single-stranded probe sequences,
- which coincide with or are complementary to the partial sequence(s) of the target DNA,
- and which are suitable for hybridization via Watson-Crick binding to the partial sequence(s) which is/are in the form of two DNA single strands of the target DNA naturally at least temporarily,
(3.) **in that** these probe sequences are coupled to the same and/or different marker molecules which can be detected by microscopy or spectroscopy or by magnetic resonance where in the case (1b) of a combination of a plurality of partial sequences or probe sequences and coupling thereof to different marker molecule(s) each unit of probe sequence and marker molecule(s) displays virtually the same binding behavior or the same melting point with the single strand, complementary thereto, of the target DNA as each of the other units,
(4.) **in that** these probe sequences are introduced into the cell and brought together with the target DNA by known methods, without using any denaturation step, in such a way that they hybridize under formation of Watson-Crick base pairing with the corresponding partial sequence(s) of the target DNA, wherein said partial sequence(s) is/are temporarily in the form of two DNA single strands,
(5.) **in that** the emitted marker signals are detected, and
(6.) **in that** the location of the target DNA on the genome is identified on the basis of the presence and/or the intensity and/or the simultaneous occurrence of different marker signals.

2. Method according to claim 1, **characterized in that** it is carried out in vivo.

3. Method according to claim 1 or 2, **characterized in that** each partial sequence has 10 to 40 nucleotides.

4. Method according to any of claims 1 to 3, **characterized in that** the marker molecules are fluorescent dyes and/or immunohistochemical marker molecules, and/or fluorescent or luminescent or magnetic nanoparticles, in particular nanocrystals (quantum dots).

5. Method according to any of claims 1 to 4 for the localization and for detecting point mutations and/or genome breakpoints and/or equivalent sequence defects, **characterized in that** in step (1) the target DNA is analyzed for a plurality of the same or different partial sequences t₁-tₙ,
that in step (3) the probe sequences S₁-Sₙ are coupled to different marker molecules which can be detected by microscopy or spectroscopy or by magnetic resonance, and
that in step (6) the absence of the marker signals of one or more probe sequences Sₓ with the simultaneous presence of the marker signals of probe sequences Sₙ-Sₓ indicates the presence of a point mutation or a genome breakpoint or an equivalent sequence defect.

6. Method according to any of claims 1 to 5, **characterized in that** the probe sequences are introduced into the cell by microinjection.

7. Use of a probe set with the characteristics:
- it comprises a single or a plurality of probe type(s) S₁-Sₙ,
- - where each probe type Sᵢ consists of an oligonucleotide,
- - - which has a length of 8 to 40 nucleotides, and
- - - which is complementary or identical to a single-stranded partial sequence tᵢ of an intracellular, double-stranded genome segment having a known nucleotide sequence - "target DNA" hereinafter -, where
the single partial sequence tᵢ or the combination of a plurality of partial sequences t₁-tₙ represents a unique pattern within the genome and does not occure outside the target DNA in the remainder of the genome within any genom-segment having the length of the target DNA,
- the probes are suitable for hybridization via Watson-Crick binding without using any denaturation step to the partial sequence(s) which is/are naturally at least temporarily in the form of two DNA single strands of the target DNA,
- and the probes are coupled to the same and/or different marker molecules which can be detected by microscopy or spectroscopy or by magnetic resonance, where in the case of a combination of a plurality of probe types and their coupling to different marker molecule(s) each unit of probe type and marker molecule(s) shows virtually the same binding behavior or the same melting point with the single strand, complementary thereto, of the target DNA as each of the other units in a FISH (fluorescence-in-situ-hybridization)-method according to claim 1.

8. Use according claim 7, **characterized in that** the oligonucleotid has 10 - 40 nucleotides.

9. Use according to claim 7 or 8, **characterized in that** the marker molecules are fluorescent dyes and/or immunohistochemical marker molecules, and/or fluorescent or luminescent or magnetic nanoparticles, in particular nanocrystals (quantum dots).

## Revendications

1. Procédé FISH (hybridation fluorescente in situ) pour l'analyse microscopique de la localisation d'un segment d'un génome natif intracellulaire sélectionné ayant une séquence de nucléotide connue - dénommée ci-après « cible ADN » -, à l'intérieur d'un génome in situ, **caractérisé**
(1.) **en ce que** la cible ADN est analysée à l'aide de bases de données de génomes sur le plan (a) d'une seule séquence partielle ou (b) de plusieurs séquences partielles de même type ou différentes, et des séquences partielles qui constituent isolément ou de manière combinée un modèle unique à l'intérieur du génome sont sélectionnées, lequel modèle ne se rencontre plus dans le reste du génome, en dehors de la cible ADN, à l'intérieur de segments quelconques du génome ayant la longueur de la cible ADN,
- chaque séquence partielle ayant une longueur comprise entre 8 et 40 nucléotides,
(2.) **en ce que** lesdites séquences partielles sont transcrites en séquences sondes complémentaires correspondantes et sont mises à disposition sous forme de séquences sondes simple brin,
- qui coïncident avec la/les séquence(s) partielle(s) de la cible ADN ou sont complémentaires de celles-ci,
- et qui conviennent, par l'intermédiaire d'une liaison Watson-Crick, pour l'hybridation sur une/des séquence(s) partielle(s) présente(s) naturellement au moins temporairement sous forme de deux simples brins d'ADN de la cible ADN,
(3.) **en ce que** ces séquences sondes sont couplées à des molécules de marquage de même type et/ou de type différent, décelables par microscopie ou spectroscopie ou par résonance magnétique,
sachant que dans le cas (1 b) d'une combinaison de plusieurs séquences partielles ou séquences sondes et de leur couplage avec différente(s) molécule(s) de marquage, chaque unité formée par une séquence sonde et une ou des molécules de marquage comporte, tout comme chacune des autres unités, pratiquement le même comportement de liaison ou le même point de fusion avec le brin simple de la cible ADN qui lui est complémentaire,
(4.) **en ce que** lesdites séquences sondes sont introduites dans la cellule au moyen de procédés connus et sont mises en contact avec la cible ADN, sans l'application d'une étape de dénaturation, de telle sorte qu'elles sont hybridées avec les séquence(s) partielle(s) de la cible ADN, présente(s) temporairement sous la forme de deux brins simples d'ADN de la cible ADN, en formant un appariement de type Watson-Crick,
(5.) **en ce que** les signaux de marquage émis sont détectés et
(6.) **en ce que** la position de la cible ADN sur le génome est identifié à l'aide de la présence et/ou de l'intensité et/ou de la formation simultanée de différents signaux de marquage.

2. Procédé selon la revendication 1, **caractérisé en ce que** ledit procédé est mis en oeuvre in vivo.

3. Procédé selon la revendication 1 ou 2, **caractérisé en ce que** chaque séquence partielle comporte 10 à 40 nucléotides.

4. Procédé selon l'une des revendications 1 à 3, **caractérisé en ce que** les molécules de marquage sont des colorants fluorescents et/ou des molécules de marquage immunohistochimiques et/ou des nanoparticules fluorescentes ou luminescentes ou magnétiques, en particulier des nanocristaux (boîtes quantiques / quantum dots).

5. Procédé selon l'une des revendications 1 à 4 pour la localisation et la détection de mutations ponctuelles et/ou de points de rupture d'un génome et/ou de défauts de séquence équivalents, **caractérisé**
**en ce que** à l'étape (1), la cible ADN est analysée sur le plan de plusieurs séquences partielles t₁-tₙ de même type ou différentes,
**en ce que** à l'étape (3), les séquences sondes S₁-Sₙ sont couplées à des molécules de marquage de type différent, décelables par microscopie ou par spectroscopie ou par résonance magnétique, et
**en ce que** à l'étape (6), l'absence de signaux de marquage d'une ou de plusieurs séquences sondes Sₓ et la présence simultanée des signaux de marquage des séquences sondes Sₙ-Sₓ indiquent la présence d'une mutation ponctuelle ou d'un point de rupture du génome ou d'un défaut de séquence équivalent.

6. Procédé selon l'une des revendications 1 à 5, **caractérisé en ce que** les séquences sondes sont introduites dans la cellule par micro-injection.

7. Utilisation d'un ensemble de sondes avec les caractéristiques :
- ledit ensemble contient un seul type de sonde ou plusieurs types de sonde S₁-Sₙ,
- - chaque type de sonde Sᵢ étant constitué d'un oligonucléotide,
- - - qui a une longueur de 8 à 40 nucléotides, et
- - - qui est complémentaire ou identique à une séquence partielle simple brin tᵢ d'un segment de génome double brin intracellulaire ayant une séquence de nucléotide connue - dénommée ci-après « cible ADN »,
l'unique séquence partielle tᵢ ou la combinaison de plusieurs séquences partielles t₁-tₙ constitue un modèle unique à l'intérieur du génome et ne se rencontre plus, en dehors de la cible ADN, dans le reste du génome à l'intérieur de segments quelconques du génome ayant la longueur de la cible ADN,
- les sondes conviennent à l'hybridation sur une/des séquence(s) partielle(s) présente(s) normalement au moins temporairement sous forme de deux brins simples d'ADN de la cible ADN par le biais de la formation de liaisons Watson-Crick, sans l'utilisation d'une étape de dénaturation,
- et les sondes sont couplées à des molécules de marquage de même type et/ou de type différent, décelables par microscopie ou par spectroscopie ou par résonance magnétique, sachant que dans le cas de la combinaison de plusieurs types de sondes et de leur couplage avec une/des molécule(s) de marquage de type différent, chaque unité formée par un type de sonde et une/des molécules de marquage a pratiquement le même comportement de liaison ou le même point de fusion avec le brin simple de la cible ADN qui lui est complémentaire, tout comme les autres unités dans un procédé FISH (hybridation fluorescente in situ) selon la revendication 1.

8. Utilisation selon la revendication 7, **caractérisée en ce que** l'oligonucléotide comporte 10 à 40 nucléotides.

9. Utilisation selon la revendication 7 ou 8, **caractérisée en ce que** les molécules de marquage sont des colorants fluorescents et/ou des molécules de marquage immunohistochimiques et/ou des nanoparticules fluorescentes ou luminescentes ou magnétiques, en particulier des nanocristaux (boîtes quantiques / quantum dots).
